Europäisches Patentamt

European Patent Office

Office européen des brevets

(19)

(11) Veröffentlichungsnummer: **0 319 767 B1**

(12) **EUROPÄISCHE PATENTSCHRIFT**

(45) Veröffentlichungstag der Patentschrift: 26.08.92

(21) Anmeldenummer: 88119388.2

(22) Anmeldetag: 22.11.88

(51) Int. Cl.5: **C07C 309/78**

(54) **Verfahren zur Herstellung von Trifluormethansulfonsäurechlorid.**

(30) Priorität: 05.12.87 DE 3741309

(43) Veröffentlichungstag der Anmeldung:
14.06.89 Patentblatt 89/24

(45) Bekanntmachung des Hinweises auf die
Patenterteilung:
26.08.92 Patentblatt 92/35

(84) Benannte Vertragsstaaten:
CH DE FR GB LI

(56) Entgegenhaltungen:
EP-A- 0 040 560
US-A- 2 277 325

JOURNAL OF THE CHEMICAL SOCIETY, Teil
III, 1955, Seiten 2901-2910; R.N. HASZELDINE
et al.: "Perfluoroalkyl derivatives of Sulphur.
Part II. Trifluoromethanesulphonic, -
sulphinic, and -sulphenic acid and the infrared spectra of compounds containing-
SO2-and S:O groups"

ANORGANIKUM, L. Kolditz, VEB Deutscher
Verlag der Wissenschaften, Berlin, 1968, S.
475, Zeile 17 - 30

(73) Patentinhaber: BAYER AG

W-5090 Leverkusen 1 Bayerwerk(DE)

(72) Erfinder: Bielefeldt, Dietmar, Dr.
Beuthener Strasse 13
W-4030 Ratingen 6(DE)
Erfinder: Marhold, Albrecht, Dr.
Carl-Duisberg-Strasse 329
W-5090 Leverkusen 1(DE)

## Beschreibung

Die vorliegende Erfindung betrifft ein Verfahren zur Herstellung von Trifluormethansulfonsäurechlorid aus Bis-(trifluormethyl)-disulfan.

Trifluormethansulfonsäurechlorid ist eine universell verwendbare Chemikalie, die beispielsweise für die Herstellung von organischen Zwischenprodukten eingesetzt wird (siehe Gmelin, Handbuch der anorganischen Chemie, Band Fluor B12, 144 (1973)).

Es sind schon Verfahren zur Herstellung von Trifluormethansulfonsäurechlorid bekannt geworden. So kann man $CF_3SCl$ mit einem Überschuß an Chlor und Wasser im Verlauf von 7 Tagen umsetzen und Trifluormethansulfonsäurechlorid erhalten (siehe US-PS 2 732 398). Man kann auch Trifluormethansulfonsäure oder deren Salze mit Phosphorpentachlorid chlorieren (siehe R.N. Haszeldine et al., J. Chem. Soc. 1955, 2901 bis 2910 und G.v. Dyke Tiers, J. Org. Chem. 28, 1244 bis 1246 (1963)). Ferner kann man Trifluormethansulfonsäurefluorid mit Hydrazin reduzieren und das entstehende Zwischenprodukt mit Chlor spalten (siehe US-PS 2 950 317) oder Trifluormethansulfonsäureanhydrid mit Kochsalz in Tetramethylensulfon umsetzen.

Nachteilig bei diesen Verfahren ist, daß sie nur mit schlechten Raum-Zeit-Ausbeuten realisiert werden können, schwierig zugängliche Ausgangsprodukte oder Reagenzien benötigen und/oder salzartige Nebenprodukte liefern, die in aufwendiger Weise entsorgt werden müssen.

Ferner ist bekannt, daß man von Trifluormethylgruppen freie Dialkyldisulfide in Abwesenheit von säure zu Sulfonylhalogeniden umsetzen kann (siehe US-PS 2 277 325) und, daß Trifluormethansulfonylchlorid hydrolyseempfindlich ist (siehe J. Chem. Soc. III, 2901-10 (1955)). Aus der EP-OS 0 040 560 ist schließlich bekannt, daß man von Trifluormethylgruppen freie Alkansulfonsäurechloride in Gegenwart von Säure in einer Emulsion herstellen kann, wenn die Tröpfchen der Emulsion eine bestimmte Größe nicht überschreiten.

Es wurde nun ein Verfahren zur Herstellung von Trifluormethansulfonsäurechlorid gefunden, das dadurch gekennzeichnet ist, daß man Bis-(trifluormethyl)-disulfan in flüssiger Phase und in Gegenwart einer starken Säure mit Chlor und Wasser umsetzt.

Das für das erfindungsgemäße Verfahren benötigte Ausgangsprodukt Bis-(trifluormethyl)-disulfan ist bekannt und auf einfache Weise zugänglich. Man erhält es beispielsweise als bisher unerwünschtes Nebenprodukt bei der Herstellung von $CF_3SCl$ (siehe W. Tullock und D.D. Coffmann, J. Org. Chem., 25, 2016 (1960)), durch Fluorierung von Trichlormethylsulfenylchlorid mit Natriumfluorid (siehe US-PS 2 884 453) oder durch Fluorierung von Bis-(trichlormethyl)-disulfan mit Fluorwasserstoff (siehe DE-OS 1 232 954 und Synthesis 6, 310 (1972)).

Die erfindungsgemäß erforderliche flüssige Phase kann beispielsweise verifiziert werden, indem man in Gegenwart eines Lösungsmittels arbeitet, das Chlor und Wasser in ausreichendem Maße zu lösen vermag und gegen eine Chlorierung inert ist. Geeignet ist hierfür beispielsweise 1,2-Dichlorethan.

Das erfindungsgemäße Verfahren wird in Gegenwart starker Säuren durchgeführt. Wenn man diese Säuren als wäßrige Säuren in entsprechender Konzentration und Menge einsetzt, so kann man damit auch das benötigte Wasser in die Reaktion einbringen. Als Säuren kommen beispielsweise in Frage: Schwefelsäure, Salpetersäure, Salzsäure, Phosphorsäure, Methansulfonsäure, Essigsäure und Fluor und/oder Chlor enthaltende organische und anorganische Säuren, wie Fluoressigsäuren, Chloressigsäuren, Perfluoralkansulfonsäuren, Fluorsulfonsäuren und Chlorsulfonsäuren. Es können auch Gemische von zwei oder mehreren Säuren eingesetzt werden.

Bevorzugt wird wäßrige Schwefelsäure in Konzentrationen von 5 bis 70 Gew.-%, wäßrige Salpetersäure in Konzentrationen von 5 bis 90 Gew.-%, wäßrige Salzsäure in Konzentrationen von 5 bis 39 Gew.-%, wäßrige Phosphorsäure in Konzentrationen von 30 bis 95 Gew.-%, wäßrige Essigsäure in Konzentrationen von 30 bis 95 Gew.-%, wäßrige Di- und/oder Trifluoressigsäure in Konzentrationen von 5 bis 100 Gew.-% und/oder wäßrige Mono-, Di- und/oder Trichloressigsäure in Konzentrationen von 5 bis 95 Gew.-%.

Das Chlor kann dem erfindungsgemäßen Verfahren in flüssiger oder gasförmiger Form zugesetzt werden, gegebenenfalls auch in Form von Verbindungen oder Gemischen, die unter den Reaktionsbedingungen des erfindungsgemäßen Verfahrens in situ Chlor liefern. Beispiele hierfür sind Natriumhypochlorit oder Chlorkalk jeweils in Verbindung mit Salzsäure.

Stöchiometrisch werden im erfindungsgemäßen Verfahren zur Umsetzung von einem Mol des Disulfans 4 Mole Wasser benötigt. Man setzt deshalb beispielsweise mindestens 3,5 Mol, vorzugsweise mindestens 4 Mol Wasser pro Mol des Disulfans ein. Nach oben hin ist die Wassermenge nicht kritisch. Man kann beispielsweise bis zu 100 Mol und mehr Wasser pro Mol des Disulfans einsetzen. Das Wasser kann dem Reaktionsgemisch als solches zugeführt werden, aber auch beispielsweise im Gemisch mit einem Lösungsmittel, z.B. 1,2-Dichlorethan und/oder in Form einer wäßrigen Säure zum Einsatz gelangen.

Stöchiometrisch werden im erfindungsgemäßen Verfahren zur Umsetzung von einem Mol des Disulfans 5 Mole Chlor benötigt. Man setzt deshalb beispielsweise mindestens 4 Mol, vorzugsweise mindestens 5 Mol Chlor pro Mol des Disulfans ein. Nach oben hin ist die Chlormenge nicht kritisch. Man kann beispielsweise bis zu 30 Mol und mehr Chlor pro Mol des Disulfans einsetzen.

Man kann beispielsweise 0,1 bis 20 Gew.-Teile Säure (berechnet als 100 %ige Säure) pro Gew.-Teil des Disulfans einsetzen.

Das erfindungsgemäße Verfahren kann man beispielsweise bei Temperaturen im Bereich von -18 bis +120°C durchführen. Bevorzugt sind Temperaturen im Bereich von 0 bis 80°C. Bei Temperaturen über 40°C ist es vorteilhaft in geschlossenen Gefäßen oder unter Druck (beispielsweise bei bis zu 100 bar) zu arbeiten, um ein Entweichen flüchtiger Bestandteile zu unterdrücken.

Das erfindungsgemäße Verfahren kann sowohl kontinuierlich als auch diskontinuierlich durchgeführt werden. Die Reaktionszeit kann beispielsweise zwischen 2 und 72 Stunden liegen.

Bei einer besonderen Ausführungsform des erfindungsgemäßen Verfahrens arbeitet man in Gegenwart eines Katalysators. Als Katalysatoren kommen vorzugsweise fluorierte Sulfonsäureamide zum Einsatz. Derartige Katalysatoren kann man beispielsweise in Mengen von 0,1 bis 20 Gew.-Teilen pro Gew.-Teil des Sulfans einsetzen.

Die Aufarbeitung kann beispielsweise so erfolgen, daß man gegebenenfalls zunächst noch vorhandenes Chlor abtrennt, z.B. durch Destillation oder Ausblasen mit Stickstoff, und dann die vorliegenden zwei Phasen trennt, z.B. durch Phasentrennung und/oder Destillation. Die abgetrennte organische Phase besteht im allgemeinen aus so reinem Trifluormethansulfonsäurechlorid, daß dieses direkt für weitere Umsetzungen verwendet werden kann.

Als Gefäßmaterialien zur Durchführung des erfindungsgemäßen Verfahrens kommen beispielsweise Stahl, Hasteloy®, Glas, Teflon® und mit Blei oder Emaille ausgekleidete Gefäße in Frage. Vorzugsweise verwendet man Glas, Teflon® oder mit Blei oder Emaille ausgekleidete Gefäße.

Das erfindungsgemäße Verfahren zeichnet sich dadurch aus, daß es von gut zugänglichen Ausgangsmaterialien ausgeht und die Herstellung von Trifluormethansulfonsäurechlorid in guten Ausbeuten und ohne die Bildung salzartiger Nebenprodukte gestattet.

Das erfindungsgemäße Verfahren gestattet die Herstellung von Trifluormethansulfonsäurechlorid auf einfache Weise, in guten bis sehr guten Reinheiten und in vorteilhaften Ausbeuten. Das erfindungsgemäße Verfahren zeichnet sich weiterhin dadurch aus, daß es bei relativ tiefen Temperaturen durchgeführt werden kann und daß es hinsichtlich Korrosionsproblemen und Energiekosten vorteilhaft ist. Außerdem gestattet es die Herstellung von Trifluormethansulfonsäurechlorid in technischem Maßstab aus einem Ausgangsprodukt, für das bisher keine technische Verwendbarkeit bekannt ist.

Es ist ausgesprochen überraschend, daß mit dem erfindungsgemäßen Verfahren die aufgezeigten Vorteile erzielt werden können, denn in J. Chem. Soc. 4230 (1954) ist beschrieben, daß beim Versuch der Oxidation von Bis-(trifluormethyl)-disulfan mit konzentrierter Salpetersäure keine Reaktion auftrat und bei hohen Temperaturen nur unidentifizierte Zersetzungsprodukte gefunden werden.

Die nachfolgenden Beispiele erläutern das erfindungsgemäße Verfahren ohne es darauf zu beschränken.

Beispiele

Beispiel 1

Zu einem Gemisch aus 500 ml konzentrierter wäßriger Salzsäure, 410 g Bis-(trifluormethyl)-disulfan und 5 g Nonafluorbutansulfonsäureamid wurde unter ständigem Rühren bei 20°C 430 g gasförmiges Chlor eingeleitet. Nach dem Ende der Chlorzugabe wurde die organische Phase von der wäßrigen Phase getrennt und mit Eiswasser gewaschen. Es wurden 317 g Trifluormethansulfonsäurechlorid (86,4 %ig) mit einem Siedepunkt von 31 bis 33°C bei 1013 mbar erhalten.

Beispiel 2

Zu einem Gemisch aus 200 ml wäßriger konzentrierter Salzsäure und 100 g Bis-(trifluormethyl-)disulfan wurden bei 40°C unter ständigem Rühren innerhalb von 21 Stunden 205 g gasförmiges Chlor eingeleitet. Nach dem Ende der Zugabe wurde die organische Phase abgetrennt und mit Eiswasser gewaschen. Es wurden 123,5 g Trifluormethansulfonsäurechlorid mit einer Reinheit von 99 % erhalten. Da Produkt kann für praktisch alle Anwendungen ohne weitere Reinigung eingesetzt werden.

Beispiel 3

Zu einem Gemisch aus 200 ml konzentrierter Salzsäure, 100 g Bis-(trifluormethyl-)disulfan und 5 g Allyl-hepta-decafluoroctansulfonsäureamid wurde bei 40°C unter ständigem Rühren innerhalb von 24 Stunden 195 g gasförmiges Chlor zudosiert. Nach dem Ende des Zudosierens wurde die organische Phase abgetrennt. Es wurden 108 g Trifluormethansulfonsäurechlorid mit einer Reinheit von 99,3 % erhalten. Dieses Produkt kann für praktisch alle Anwendungen ohne weitere Reinigung verwendet werden.

### Beispiel 4

In einem Autoklaven aus Teflon® wurden 50,5 g Bis-(trifluormethyl-)disulfan, 57 ml flüssiges Chlor und 5 g Nonafluorbutansulfonsäureamid in 250 ml konzentrierter wäßriger Salzsäure gelöst. Das Gemisch wurde auf 120°C erhitzt und 8 Stunden bei dieser Temperatur belassen. Nach der Phasentrennung wurde als organische Phase 57 g Trifluormethansulfonsäurechlorid mit einer Reinheit von 99 % erhalten.

### Patentansprüche

1. Verfahren zur Herstellung von Trifluormethansulfonsäurechlorid, dadurch gekennzeichnet, daß man Bis-(trifluormethyl-)disulfan in flüssiger Phase und in Gegenwart einer starken Säure mit Chlor und Wasser umsetzt.

2. Verfahren nach Anspruch 1, dadurch gekennzeichnet, daß man in Gegenwart eines Lösungsmittels arbeitet.

3. Verfahren nach Ansprüchen 1 und 2, dadurch gekennzeichnet, daß man in Gegenwart von Schwefelsäure, Salpetersäure, Salzsäure, Phosphorsäure, Methansulfonsäure, Essigsäure und/oder Fluor und/oder Chlor enthaltenden organischen oder anorganischen Säuren arbeitet.

4. Verfahren nach Ansprüchen 1 bis 3, dadurch gekennzeichnet, daß man pro Mol Bis-(trifluormethyl-)disulfan mindestens 3,5 Mol Wasser einsetzt.

5. Verfahren nach Ansprüchen 1 bis 4, dadurch gekennzeichnet, daß man pro Mol Bis-(trifluormethyl-)disulfan mindestens 4 Mol Chlor einsetzt.

6. Verfahren nach Ansprüchen 1 bis 5, dadurch gekennzeichnet, daß man es bei Temperaturen im Bereich von -18 bis +120°C durchführt.

7. Verfahren nach Ansprüchen 1 bis 6, dadurch gekennzeichnet, daß man es in Gegenwart fluorierter Sulfonsäureamide als Katalysator durchführt.

8. Verfahren nach Ansprüchen 1 bis 7, dadurch gekennzeichnet, daß man es bei Drucken von 1 bis 100 bar durchführt.

9. Verfahren nach Ansprüchen 1 bis 8, dadurch gekennzeichnet, daß man das Reaktionsgemisch durch Phasentrennung und/oder Destillation aufarbeitet.

### Claims

1. Process for the preparation of trifluoromethanesulphonyl chloride, characterised in that bis-(trifluoromethyl)-disulphane is reacted with chlorine and water in the liquid phase and in the presence of a strong acid.

2. Process according to Claim 1, characterised in that the reaction is carried out in the presence of a solvent.

3. Process according to Claims 1 and 2, characterised in that the reaction is carried out in the presence of sulphuric acid, nitric acid, hydrochloric acid, phosphoric acid, methanesulphonic acid, acetic acid and/or organic or inorganic acids containing fluorine and/or chlorine.

4. Process according to Claims 1 to 3, characterised in that at least 3.5 moles of water are employed per mole of bis-(trifluoromethyl)-disulphane.

5. Process according to Claims 1 to 4, characterised in that at least 4 moles of chlorine are employed per mole of bis-(trifluoromethyl)-disulphane.

6. Process according to Claims 1 to 5, characterised in that it is carried out at temperatures in the range from -18 to + 120°C.

7. Process according to Claims 1 to 6, characterised in that it is carried out in the presence of fluorinated sulphonamides as catalyst.

8. Process according to Claims 1 to 7, characterised in that it is carried out at pressures of 1 to 100 bar.

9. Process according to Claims 1 to 8, characterised in that the reaction mixture is worked up by phase separation and/or distillation.

### Revendications

1. Procédé de préparation du chlorure de l'acide trifluorométhane-sulfonique, caractérisé en ce que l'on fait réagir le bis-(trifluorométhyl)-disulfane en phase liquide et en présence d'un acide fort avec le chlore et l'eau.

2. Procédé selon la revendication 1, caractérisé en ce que l'on opère en présence d'un solvant.

**3.** Procédé selon les revendications 1 et 2, caractérisé en ce que l'on opère en présence de l'acide sulfurique, de l'acide nitrique, de l'acide chlorhydrique, de l'acide phosphorique, de l'acide méthane-sulfonique, de l'acide acétique et/ou d'un acide organique ou minéral contenant du fluor et/ou du chlore.

**4.** Procédé selon les revendications 1 à 3, caractérisé en ce que l'on utilise au moins 3,5 mol d'eau par mole du bis-(trifluorométhyl)-disulfane.

**5.** Procédé selon les revendications 1 à 4, caractérisé en ce que l'on utilise au moins 4 mol de chlore par mole du bis-(trifluorométhyl)-disulfane.

**6.** Procédé selon les revendications 1 à 5, caractérisé en ce que l'on opère à des températures dans l'intervalle de -18 à +120°C.

**7.** Procédé selon les revendications 1 à 6, caractérisé en ce que l'on opère en présence d'un sulfonamide de fluoré qui sert de catalyseur.

**8.** Procédé selon les revendications 1 à 7, caractérisé en ce que l'on opère à des pressions de 1 à 100 bar.

**9.** Procédé selon les revendications 1 à 8, caractérisé en ce que l'on traite le mélange de réaction par séparation de phases et/ou distillation.